# FASCICULE DE BREVET EUROPEEN

(11) **EP 0 596 342 B1**
(45) Date de publication et mention de la délivrance du brevet: **23.07.1997**
(21) Numéro de dépôt: 93117112.8
(22) Date de dépôt: 22.10.1993
(51) Int. Cl.: G01S 15/18, G01N 29/10, A61B 8/08

(54) **Appareil de mesure par ultrasons minimisant le nombre de valeurs numériques traitées par les moyens de traitement**
Ultraschallmessgerät mit Minimierung der Zahl der durch die Verarbeitungsmitteln verarbeiteten digitalen Daten
Ultrasonic measuring apparatus which minimizes the number of digital values treated by processing means

(30) Priorité: 02.11.1992 FR 9213351
(43) Date de publication de la demande: 11.05.1994
(73) Titulaire: ASULAB S.A., CH-2501 Bienne (CH)
(72) Inventeur: Mignot, Jean-Pierre, CH-2034 Peseux (CH)
(74) Mandataire: de Montmollin, Henri

(56) Documents cités:
- EP-A- 0 324 855
- EP-A- 0 356 629
- US-A- 4 324 141
- MEDICAL AND BIOLOGICAL ENGINEERING AND COMPUTING vol. 25, no. 2, Mars 1987, Stevenage, GB, p 189-194;, M. ERIKSEN: 'Noninvasive measurement of arterial diameters in humans using ultrasound echoes with prefiltered waveforms'

## Description

La présente invention concerne un appareil de mesure par ultrasons qui émet une impulsion ultrasonore en direction d'un objet ayant des parois, qui reçoit des échos réfléchis à partir de ces parois, et qui traite ultérieurement des signaux créés en réponse à ces échos afin de déterminer la position temporelle desdites parois.

L'invention trouve des applications dans le domaine médical et peut être utilisée par exemple pour suivre l'évolution temporelle de la position des parois antérieure et postérieure d'un vaisseau sanguin pour déterminer des variations du diamètre intérieur et les variations de l'épaisseur des parois du vaisseau sanguin en fonction du temps. Bien que cette invention soit décrite en liaison avec cet exemple d'application, on comprendra que l'invention n'est pas limitée à cette application et que l'invention peut être, par exemple, utilisée pour la mesure de l'épaisseur de la lentille cornéenne ou pour la mesure non-invasive d'autres organes.

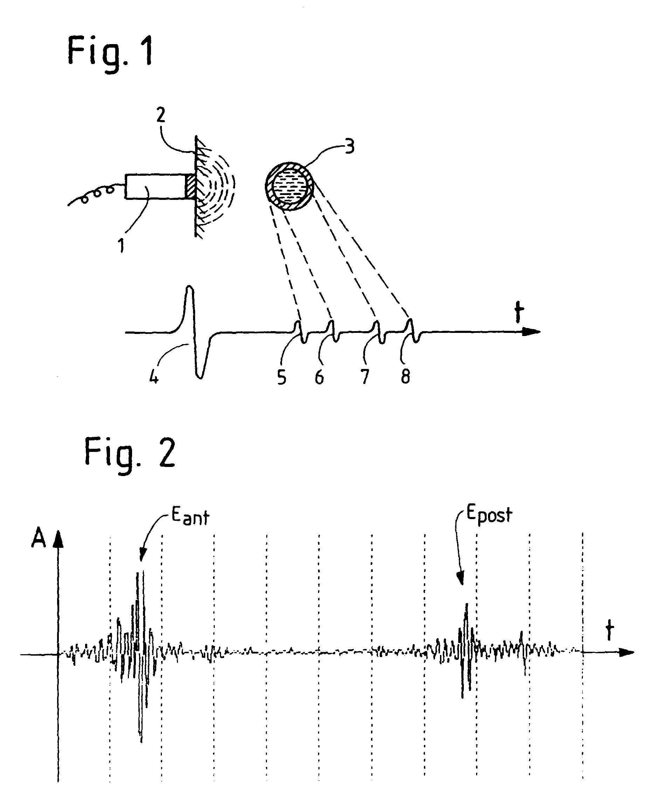

La figure 1 illustre schématiquement le principe connu de la mesure de la position d'une paroi mobile d'un vaisseau sanguin. Cette figure représente un transducteur ultrasonore 1 placé sur la peau 2 d'un sujet en face d'une artère 3 représentée en coupe transversale. Le transducteur 2 est commandé par un circuit électronique pour émettre une impulsion ultrasonore 4 et pour recevoir les échos résultant de la réflexion de cette impulsion sur les interfaces artère-tissu et artère-sang et pour créer un signal d'écho en réponse à celle-ci. Selon la fréquence du transducteur ultrasonore, on peut détecter quatre échos distincts 5, 6, 7 et 8, ou seulement deux échos correspondant respectivement à une combinaison des échos 5 et 6 et à une combinaison des échos 7 et 8.

On détermine le mouvement de chaque interface de la manière suivante. Le transducteur 1 émet une impulsion 4 avec une fréquence de répétition comprise généralement entre 10 Hz et 5 kHz. Pour suivre la position des échos, qui sont détectés par le transducteur, dont le retard dépend de la position de chaque interface, on utilise une fenêtre temporelle de largeur fixée qui définit un intervalle de temps dans lequel les échos sont attendus, et qui est ajustée après chaque cycle de sorte que les échos soient détectés au centre de cette fenêtre si l'interface est immobile.

La connaissance de la position temporelle de chaque interface ainsi que la vitesse de propagation de l'impulsion dans le sang et dans les tissus permettent en mesurant l'intervalle de déterminer l'évolution du diamètre intérieur et l'évalution de l'épaisseur des parois antérieure et postérieure du vaisseau sanguin 3 en fonction du temps.

La figure 1 est un schéma de principe. En pratique, les échos résultant de la réflexion d'une impulsion sur les parois antérieure et postérieure du vaisseau sanguin 3 ne sont pas aussi purs, mais présentent une forme beaucoup plus complexe comme cela est représenté par les échos élémentaires Eₐₙₜ et Eₚₒₛₜ à la figure 2. Cette déformation résulte de ce que le signal ultrasonore traverse des tissus ayant des caractéristiques différentes et de ce que l'interface entre la paroi d'un vaisseau sanguin et le tissu environnant n'est pas parfaitement définie, à la différence par exemple d'une interface entre une plaque de métal et l'air.

La position de l'interface engendrant ces échos, notamment dans le domaine médical, ne peut ainsi pas être directement et automatiquement déterminée à partir de la forme du signal d'écho.

Plusieurs techniques de détection de la position d'une interface mobile sont connues.

Selon une première technique, la position d'une interface est déterminée manuellement. L'utilisateur affiche le signal d'écho sur un oscilloscope ou d'autres moyens d'affichage et choisit un point particulier sur le signal d'écho sur lequel le suiveur d'écho doit être verrouillé. Malheureusement, cette technique nécessite une grande expérience de la part de l'utilisateur pour déterminer le point particulier de l'écho élémentaire qui correspond à la position de l'interface. En pratique, l'utilisateur choisit soit le pic ayant la plus grande amplitude ou le pic central de l'écho élémentaire. Il n'y a aucune assurance que le point choisi corresponde effectivement à la position de l'interface.

Selon une deuxième technique, on traite le signal d'écho afin de supprimer le bruit en ne gardant uniquement la partie du signal résultant de la réflexion du signal ultrasonore sur chaque interface. Cette technique, présente toutefois l'inconvénient que le signal numérique traité ne peut pas être réalisé en temps réel. En fait avec les moyens de calcul actuellement disponibles le traitement des signaux reçus pendant l'ouverture de la fenêtre temporelle prend 0,1 à 5 secondes alors que la fréquence de répétition de l'impulsion est de 500 Hz, le temps nécessaire pour traiter le signal d'écho, et réaliser les opérations associées telles que la lecture et la commande des dispositifs périphériques est de l'ordre de 0,002 secondes.

Il est donc nécessaire de procéder en deux étapes: dans un premier temps, on numérise et on mémorise en temps réel les signaux d'écho à étudier et dans un second temps, on traite ces signaux d'écho. On remarque que cette technique présente trois inconvénients et qui sont la nécessité de disposer d'un volume de mémoire important, la durée de traitement des signaux d'écho et l'absence de contrôle en temps réel des données en cours d'acquisition.

Le brevet européen No 534 217 publié le 31 Mars 1993 décrit un appareil de mesure par ultrasons qui vise à résoudre les problèmes soulevés par cette deuxième technique. Cet appareil émet une première impulsion ultrasonore en direction d'un vaisseau sanguin, convertit le signal d'écho créé à partir des échos détectés par le transducteur en une série de valeurs numériques (créées pendant l'ouverture de la fenêtre temporelle) qui sont alors stockées. Dans une étape d'initialisation, ces valeurs numériques stockées sont traitées afin de sélectionner un point de référence dans chaque écho élémentaire du signal d'écho, on détermine pour chaque écho élémentaire la position temporelle de chaque interface produisant l'écho élémentaire et on calcule pour chaque écho élémentaire l'intervalle temporelle entre la position du point de référence de cet écho élémentaire et la position temporelle de l'interface obtenu par le traitement.

En parallèle avec ce traitement, une étape d'assimilation se produit dans laquelle les valeurs numériques résultant de la détection des signaux d'écho provenant des impulsions ultrasonores ultérieures sont traitées afin de suivre la position de la position temporelle des points de référence provenant de chaque signal d'écho.

Il s'ensuit une phase d'acquisition dans laquelle la position temporelle de chaque interface, correspondant à chaque écho élémentaire de chaque signal d'écho résultant des impulsions ultrasonores ultérieures à la phase d'assimilation, est suivie et mémorisée. Enfin, une étape d'exploitation au cours de laquelle les données mémorisées à l'étape d'acquisition est utilisée pour fournir des informations à l'utilisateur tel que l'affichage du diamètre du vaisseau sanguin en fonction du temps.

L'homme du métier comprendra que d'autres techniques peuvent être utilisées en liaison avec la présente invention pour déterminer la position d'une interface à l'aide de signaux d'écho.

Alors que la technique décrite dans le brevet européen No 534 217 évite la nécessité de traiter chaque signal d'écho numérisé pour déterminer la position de chaque interface dans chaque écho élémentaire de chaque signal d'écho en temps réel, un traitement de chaque signal d'écho numérisé entre les impulsions ultrasonores consécutives est néanmoins nécessaire.

Selon cette technique chaque signal d'écho, numérisé pendant l'ouverture de la fenêtre temporelle, nécessite d'être traité afin de suivre les points de référence sélectionnés dans les échos élémentaires. Afin de permettre la réalisation de ce suivi, chaque point de référence doit tomber dans la partie du signal d'écho numérisé pendant l'ouverture de la fenêtre temporelle.

L'affichage de chaque signal d'écho reçu peut être fourni à la fois avant et pendant les étapes d'initialisation, d'assimilation, et de traitement de sorte que l'utilisateur peut vérifier le positionnement correct du transducteur sur la peau du sujet et contrôler la forme des signaux d'écho. Des moyens de traitement contrôlent cet affichage en temps réel. Chaque valeur numérique correspondant à chaque signal d'écho nécessite d'être lue à partir d'une adresse-mémoire et d'être ajustée entre les caractéristiques tels que le retard et de décalage. Les moyens de traitement doivent également créer une courbe de meilleur ajustement pour ces valeurs numériques.

Un dispositif périphérique tel qu'un clavier peut être également utilisé pour fournir un contrôle d'utilisateur sur l'affichage de chaque signal d'écho et sur toute information graphique associée qui est également affichée. Les moyens de traitement doivent fournir le contrôle de clavier pour permettre l'entrée de signaux devant être lus à partir d'un tel dispositif. D'autres équipements de mesure tel qu'un sphygmomanomètre, un plethysmographe ou un détecteur Doppler peuvent être reliés au moyens de traitement. Les signaux provenant de cet équipement peuvent nécessiter d'être échantillonnés, lus et affichés en même temps avec chaque signal d'écho reçu à partir du transducteur ultrasonore. En outre, des moyens de traitement doivent contrôler toutes les fonctions associées avec la mémoire dans laquelle les valeurs numériques sont stockées.

Il est également nécessaire de prévoir un certain temps entre deux impulsions consécutives pour réaliser les calculs nécessaires pour chacune des étapes d'initialisation d'assimilation et de traitement.

Le temps nécessaire pour réaliser le traitement décrit ci-dessus est fonction de la vitesse des moyens de traitement et du nombre de valeurs numérisées à partir de chaque signal d'écho pendant l'ouverture de la fenêtre temporelle. La vitesse à laquelle un signal d'écho est échantillonné afin de permettre sa reconstruction précise est déterminée par la fréquence fondamentale du signal d'écho. Dans le cas de signaux d'écho résultant de la réflexion d'impulsions non ultrasonores provenant de vaisseaux sanguins telle qu'une fréquence d'échantillonnage peut être de 100 MHz c'est-à-dire que le signal d'écho est échantillonné toutes les 10 nanosecondes.

Une artère humérale peut avoir un diamètre aussi faible que 4 mm. Les échos provenant des parois d'une artère humérale se produiront alors relativement rapidement les uns après les autres. Il s'est révélé qu'un signal d'écho résultant d'une telle artère nécessite au moins de l'ordre de 500 valeurs numériques à une fréquence d'échantillonnage de 100 MHz pour permettre une reconstruction précise. Toutefois, une artère fémorale ou carotide peuvent avoir un diamètre maximum de 1 cm. Les échos provenant de ces parois seront alors plus écartés les uns des autres et plus de 1500 valeurs numériques peuvent être nécessaires pour reconstruire de façon précise le signal d'écho correspondant.

Les moyens de traitement utilisés pour traiter ces valeurs numérisées peuvent être fournis par un microprocesseur, le plus puissant que l'on peut obtenir actuellement fonctionne à 32 MHz. Le temps nécessaire pour un cycle d'instruction (généralement environ 10 cycles de machine) dans de tels microprocesseurs est d'environ 0,4 msec, et plusieurs cycles d'instruction sont nécessaires pour traiter chaque valeur numérique de la façon décrite plus haut. Si l'impulsion ultrasonore produite par le transducteur est répétée à une fréquence de 500 Hz, moins de 6000 cycles d'instruction peuvent être réalisés entre des impulsions consécutives.

Il ressort que pour des grosses artères, les microprocesseurs existants ne sont pas capables de traiter un nombre suffisant de valeurs numériques provenant d'un signal d'écho dans le temps s'écoulant entre des impulsions consécutives comprenant entre elles les échos élémentaires produits par les deux parois de l'artère. Le suivi des points de référence des signaux d'échos, l'affichage des signaux d'écho, le contrôle de l'utilisateur nécessaire et le traitement du signal d'écho ne peut ainsi pas être réalisé de façon correcte. Alors qu'un ordinateur plus puissant pourrait être utilisé dans ces applications, le surcoût de tels ordinateurs est prohibitif.

L'invention a donc pour but de fournir un appareil de mesure par ultrasons qui palie les inconvénients de l'art antérieur.

A cet effet, l'invention a donc pour objet un appareil de mesure par ultrasons comprenant un transducteur ultrasonique pour émettre une impulsion ultrasonore à une fréquence de répétition prédéterminée vers un objet ayant une pluralité de parois, pour recevoir des échos réfléchis à partir desdites parois et pour produire un signal d'écho ayant une pluralité de composants d'échos élémentaires, des moyens de numérisation pour numériser ledit signal d'écho en une série de valeurs numériques, une mémoire-tampon pour stocker lesdites valeurs numériques provenant desdits moyens de numérisation, des moyens de contrôle pour transférer lesdites valeurs numériques provenant desdits moyens de numérisation dans ladite mémoire-tampon, des moyens de mémorisation pour stocker lesdites valeurs numériques provenant de ladite mémoire-tampon et des moyens de traitement pour transférer ladite série de valeurs numériques stockées dans ladite mémoire-tampon dans lesdits moyens de mémorisation entre des impulsions consécutives, caractérisé en ce que lesdits moyens de traitement sont, en outre, aptes à prélever à partir desdites valeurs numériques, à des fins de non traitement, un groupe desdites valeurs numériques numérisées entre des composants d'échos élémentaires consécutifs devant être traités entre des impulsions consécutives.

Le nombre de valeurs numériques provenant de chaque signal d'écho qui doit d'être traité en temps réel entre des impulsions ultrasonores est ainsi diminué et la gamme de diamètres de vaisseaux sanguins qui peut être mesurée par les appareils existants est élargie de façon correspondante.

La description suivante se réfère plus en détail à des caractéristiques de l'appareil de mesure par ultrasons de la présente invention. Afin de faciliter la compréhension de la présente invention, on se référera dans la description aux dessins annexés dans lesquels un appareil de mesure par ultrasons est illustré dans un mode de réalisation préféré. On comprendra bien entendu que l'appareil de mesure par ultrasons de la présente invention n'est pas limité au mode de réalisation préféré illustré aux dessins dans lesquels :
- la figure 1 déjà décrite, représente le principe de la mesure par ultrasons de la position d'interface des parois antérieure et postérieure d'un vaisseau sanguin;
- la figure 2, déjà décrite, représente la forme temporelle d'un signal d'écho représentant deux échos élémentaires produits par les parois antérieure et postérieure d'un vaisseau sanguin;
- la figure 3 montre schématiquement un mode de réalisation d'un appareil de mesure par ultrasons selon la présente invention;
- la figure 4 montre la représentation des valeurs numériques correspondant à un signal d'écho reçu par l'appareil de la figure 3; et
- la figure 5 montre la représentation de la figure 4 après qu'un groupe de valeurs numériques se trouvant entre des échos élémentaires aient été retirés.

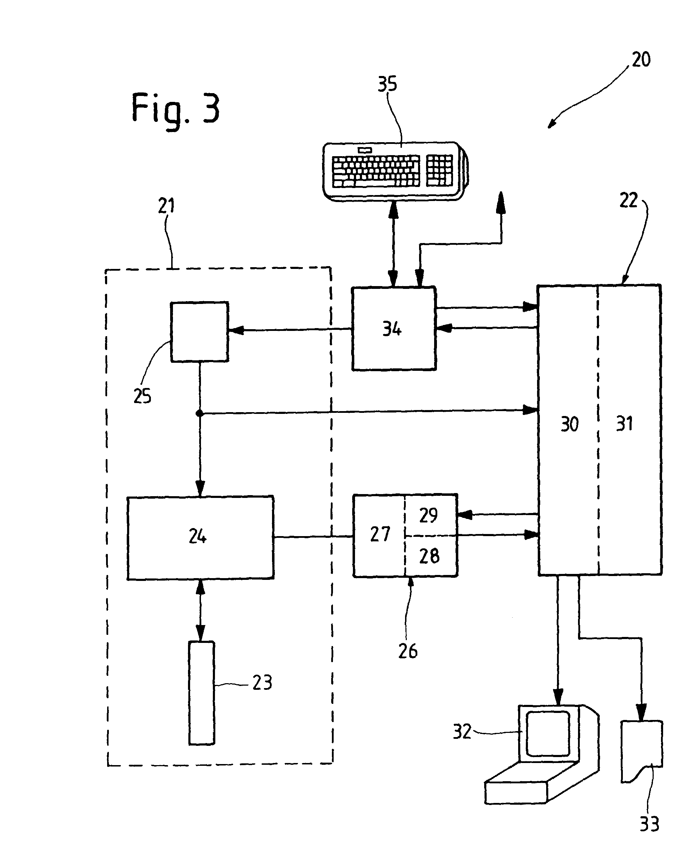

On a représenté schématiquement à la figure 3 un appareil de mesure par ultrasons 20 comprenant un transducteur ultrasonore 21 et un dispositif de calcul 22. Le transducteur ultrasonore 21 comporte une sonde ultrasonore 23 pour émettre un signal ultrasonore et recevoir les échos résultant de la réflexion de cette onde ultrasonore, un circuit de commande 24 pour commander la sonde ultrasonore 23 et une horloge 25. Cette dernière délivre au circuit de commande 24 un signal définissant la fréquence de répétition du signal d'interrogation émis par la sonde ultrasonore 23.

Le circuit de commande 24 comprend un circuit d'émission délivrant une impulsion électrique qui est transformée par la sonde ultrasonore 23 en un signal ultrasonore correspondant et un circuit de réception recevant le signal électrique délivré par la sonde ultrasonore correspondant au signal d'écho ultrasonore reçu par la sonde 23. Le circuit de commande 24 et la sonde ultrasonore 23 sont de type classique. La fréquence centrale de la pulsion ultrasonore est choisie en fonction de l'application visée. Elle est par exemple de 2 à 20 MHz.

Le signal électrique d'écho délivré par le circuit de commande 24 est reçu par le dispositif de calcul 22 à travers un convertisseur analogique-numérique 26. Pour celui-ci, on peut utiliser un produit du type STR 8100 de la société SONIX inc. (Springfield, VA, USA) qui est un convertisseur analogique-numérique 8 bits pouvant traiter jusqu'à 10⁸ échantillons/seconde. Le convertisseur analogique-numérique comprend des moyens de numérisation 27, une mémoire-tampon 28 et un processeur 29. Les moyens de numérisation 27 convertissent le signal d'écho analogique reçu provenant du circuit de commande en une série de valeurs numériques qui sont stockées dans la mémoire-tampon 28. Le processeur 29 commande le fonctionnement les moyens de numérisation 27, le stockage des valeurs numériques dans la mémoire-tampon 28 et le transfert des valeurs numériques stockées vers le dispositif de calcul 22.

Le dispositif de calcul 22 comporte un suiveur d'échos qui est utilisé de manière classique pour suivre la position temporelle de chaque signal d'écho élémentaire du signal d'écho par rapport au signal ultrasonore émis. Cette position temporelle c'est-à-dire finalement le retard de chaque signal d'écho élémentaire sur l'impulsion ultrasonore émise varie avec la distance entre la sonde ultrasonore et les parois mobiles sur lesquelles se réfléchit l'impulsion ultrasonore. Pour réaliser ce suivi, le suiveur d'écho du dispositif de calcul 22 reçoit le signal d'horloge produit par l'horloge 25 et délivre au convertisseur analogique-numérique 26 un signal de retard pour débuter la numérisation du signal d'écho à un instant adéquat. Le suiveur d'écho est de préférence de type détection de l'extremum (positif ou négatif) du signal d'écho numérisé. Cet extremum n'est pas la valeur correcte pour apprécier le mouvement des parois mobiles car la distance entre deux points d'échantillonnage est égale à c/(2.f), où c = 1540 m/s (la vitesse de l'onde ultrasonore dans le milieu) et f=100 MHz est la fréquence d'échantillonnage. Il permet seulement de suivre grossièrement le déplacement de l'écho.

Selon une variante, le suiveur d'écho pourrait être de type détection du passage par zéro tel que décrit dans les documents EP-A-337 297 et EP-A-356 629.

Le dispositif de calcul 22 met en oeuvre le procédé de mesure selon l'invention pour déterminer la position temporelle des interfaces du vaisseau sanguin 3. A cet effet il comporte principalement des moyens de traitement 30 et des moyens de mémorisation 31. Les moyens de traitement 30 sont avantageusement un ordinateur personnel à processeur des familles de type 80X387 ou 80X486. Divers équipements périphériques peuvent être ajoutés tels que de moyens d'affichage 32, des moyens d'impression 33 et un circuit d'entrée/sortie 34, ce dernier permettant à l'utilisateur de contrôler le fonctionnement de l'appareil de mesure 20 par l'intermédiaire d'un clavier 35 ou d'un autre dispositif d'entrée d'utilisateur. Ce circuit d'entrée/sortie 34 peut être également relié à l'horloge 25 pour contrôler la fréquence de répétition du signal d'horloge provenant du dispositif de calcul 22. Il peut également servir pour synchroniser d'autres équipements de mesure tel qu'un sphygmomanomètre, un pléthysmographe ou une sonde Doppler afin de mesurer la pression sanguine et la vitesse de sang.

Le fonctionnement de l'appareil de mesure par ultrasons 20 pour déterminer le diamètre intérieur en fonction du temps et l'épaisseur des parois du vaisseau sanguin 3 va maintenant être décrit.

Avant de commencer les mesures en tant que telles, l'utilisateur sélectionne les paramètres de l'appareil, tels que la fréquence de répétition et le type de détecteur, c'est-à-dire la fréquence centrale de l'impulsion ultrasonore. Ces paramètres peuvent être sélectionnés par l'intermédiaire du clavier 35 ou automatiquement par les moyens du traitement 30 en fonction de l'application choisie par l'utilisateur. Par exemple, dans le cas de la mesure de diamètre interne et de l'épaisseur d'une paroi d'un vaisseau sanguin, la fréquence de répétition est de l'ordre de 10 Hz à 5 kHz pour la mesure de l'artère carotide. La durée du retard émis vers le convertisseur analogique-numérique 26 est également ajustée, soit manuellement soit automatiquement de sorte que les échos élémentaires des signaux d'échos puissent être placés à l'intérieur de la fenêtre temporelle et que chaque écho soit correctement suivi par le suiveur d'échos.

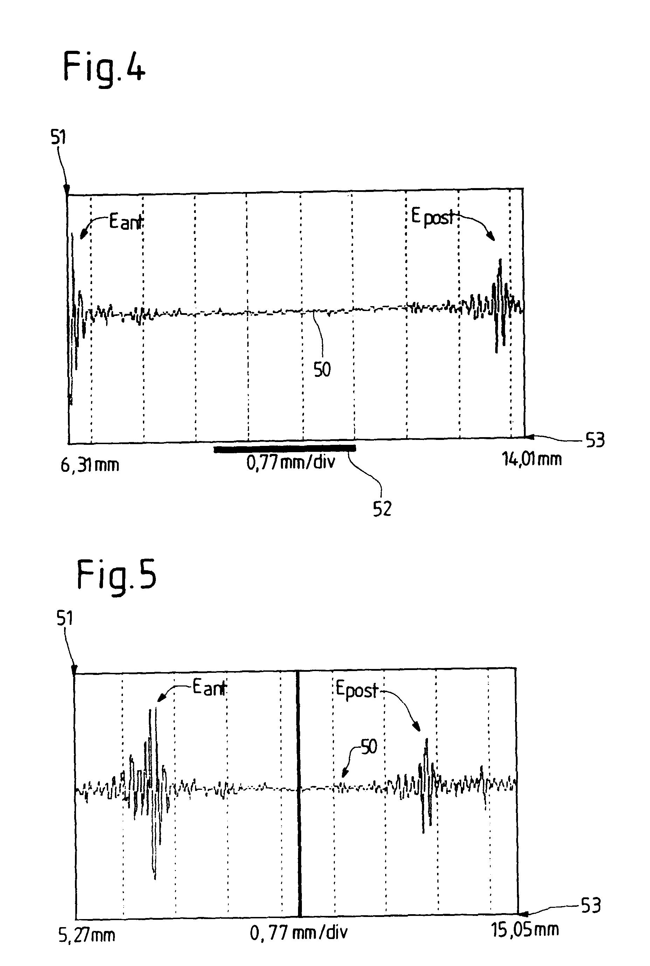

Les moyens d'affichage 32 peuvent être utilisés pour aider l'utilisateur dans la sélection de ces paramètres. Comme cela ressort de la figure 4, une trace d'un signal d'écho reçu par le convertisseur analogique-numérique 26 est présentée à l'utilisateur pour l'aider à positionner de façon correcte le transducteur ultrasonore 21 par rapport au vaisseau sanguin 3. Lorsque le gain horizontal de la trace 50 est ajusté de façon appropriée, chacune des valeurs numériques à l'intérieur d'une gamme qui peut être traitée par le dispositif de calcul 22 entre des impulsions consécutives peut être affichée. En fonction de la trace 50, l'utilisateur peut ajuster le retard à partir de l'émission de l'impulsion ultrasonore jusqu'à ce que le convertisseur analogique-numérique 26 débute la numérisation du signal d'écho, définissant ainsi l'axe vertical gauche 51 de la trace 50.

Si le temps entre la détection d'échos élémentaires provenant de la paroi antérieure et de la paroi postérieure du vaisseau sanguin 3 est trop grand, l'un ou l'autre des échos élémentaires Eₐₙₜ et Eₚₒₛₜ comme cela est partiellement visible sur la trace 50, peut tomber en dehors de la gamme des valeurs numériques qui peut être traitée par le dispositif de calcul 22 entre des impulsions consécutives (comme cela est illustré à la figure 4). Pour résoudre ce problème, l'appareil 20 est apte à permettre à l'utilisateur de sélectionner un groupe de valeurs numériques qui ne sont pas traitées. Une ligne 52 est affichée sur les moyens d'affichage 32 le long de l'axe horizontal 53 de la trace 50 laquelle ligne permet la visualisation du groupe de valeurs numériques devant être coupées de la trace 50. La longueur de la ligne 52 peut être ajustée par l'utilisateur.

En choisissant de façon appropriée la longueur de la ligne 52 et le retard jusqu'à ce que le convertisseur analogique-numérique débute la numérisation des signaux d'échos, une partie désirée de la trace 50 peut être retirée. On comprendra que l'affichage de la trace 50 et de la ligne 52 représente simplement une façon pratique selon laquelle l'utilisateur peut choisir un groupe de valeurs numériques à partir des signaux qui ne doivent pas être traités, sur la base de la séparation entre les échos élémentaires et des signaux d'échos.

Une fois que la partie de la trace 50 devant être retirée à été choisie, des informations identifiant cette partie choisie sont envoyées à partir du dispositif de calcul 22 vers le processeur 29 du convertisseur analogique numérique 26. Après le retard prédéterminé qui suit l'émission de chaque impulsion ultrasonore, les moyens de numérisation 27 commencent à numériser le signal d'écho reçu provenant du circuit de commande 24. Chaque valeur numérique ainsi créée est stockée par le processeur 29 dans un endroit séparé à l'intérieur de la mémoire-tampon 28. A la valeur numérique correspondant au début de la partie devant être retirée de la trace 50, le processeur 29 cesse son stockage de façon temporaire. Pendant cette cessation temporaire, lesdits moyens de numérisation continuent de numériser le signal d'écho mais les valeurs numériques ainsi produites ne sont pas stockées. A la valeur numérique correspondant à la fin de la partie devant être retirée, le processeur 29 mémorise à nouveau les valeurs numériques provenant desdits moyens de numérisation 27 dans des emplacements de mémoire séparés dans la mémoire-tampon 28. La fenêtre temporelle pendant laquelle les échos sont attendus demeure ainsi ouverte pendant un temps total fixé mais cette fermeture effective et cette réouverture de la fenêtre permet le positionnement des échos élémentaires Eₐₙₜ et Eₚₒₛₜ à l'intérieur de la fenêtre temporelle.

Une fois que le nombre maximum de valeurs numériques provenant de chaque signal d'écho qui peut être traité par le dispositif de calcul 22 a été stocké, ces valeurs numériques sont transférées dans le dispositif de calcul 22 et stockées dans les moyens de mémorisation 31. Les moyens de traitement 30 entraînent alors l'affichage par les moyens d'affichage 32 des valeurs numériques stockées dans les moyens de mémorisation 31 comme cela est représenté à la figure 5. On peut voir que par la sélection appropriée de la partie devant être retirée de la trace 50 et le retard jusqu'à ce que commence la numérisation, les valeurs numériques correspondant à la fois aux échos élémentaires Eₐₙₜ et Eₚₒₛₜ peuvent être incluses dans ces valeurs numériques qui peuvent être traitées par le dispositif de calcul 22. La forme des signaux d'échos peut être ainsi vérifiée et contrôlée pendant le fonctionnement de l'appareil 20. Le suiveur d'échos permet également de fonctionner de façon correcte et suit de façon automatique les points de référence sur les signaux d'échos pendant que le procédé de mesure est mis en oeuvre par l'appareil 20.

Selon une variante, les valeurs numériques crééent pas les moyens de numérisation 27 provenant des signaux d'échos peuvent être stockées dans la mémoire-tampon 100 un groupe sélectionné de valeurs numériques entre les échos élémentaires étant tout d'abord retiré. Les moyens de traitement 30 peuvent être aptes à ne pas transférer ce groupe sélectionné quand d'autres valeurs numériques sont transférées de la mémoire-tampon 28 vers les moyens de mémorisation 31. Ce transfert plus compliqué limiterait toutefois le temps disponible aux moyens de traitement 30 entre des impulsions consécutives pendant lequel les valeurs numériques doivent être traitées.

Selon une autre variante, on peut simplement stocker les valeurs numériques reçues provenant des moyens de numérisation 27 dans la mémoire-tampon 28 et transférer ces valeurs numériques de la mémoire-tampon vers les moyens de mémorisation 31 sans retirer un groupe de valeurs numériques entre les échos élémentaires. Les moyens de traitement 30 peuvent dans ce cas être aptes à ignorer de façon effective un groupe sélectionné de valeurs numériques qui ne sont pas à traiter. Toutefois, le temps nécessaire pour le traitement entre deux impulsions consécutives est à nouveau réduit en raison du nombre augmenté de valeurs numériques qu'il est nécessaire de transférer et la façon la plus compliquée et par conséquent la plus lente dans laquelle les valeurs numériques ainsi stockées dans les moyens de mémorisation 31 doivent être traitées.

L'utilisateur peut alors procéder à la mesure de la position de l'interface d'un vaisseau sanguin en utilisant l'appareil 20 pour mettre en oeuvre le procédé de mesure décrit dans la demande de brevet suisse No 2871/91. Ce procédé de mesure ne sera pas décrit ici en détail mais peut être divisé en trois étapes à savoir une étape d'initialisation, une étape d'acquisition et une étape de traitement. Dans l'étape d'initialisation, les valeurs numériques transférées et stockées dans les moyens de mémorisation 31 sont traitées afin de sélectionner un point de référence dans chaque écho élémentaire du signal d'écho déterminent, pour chaque écho élémentaire, la position temporelle de chaque interface produisant cette écho élémentaire et calculent, pour chaque écho élémentaire, l'intervalle temporel entre la position du point de référence de cet écho élémentaire et la position temporelle de l'interface obtenu par le traitement.

En parallèle à ce traitement, la phase d'acquisition se déroule et au cours de laquelle les valeurs numériques résultant de la détection de signaux d'échos provenant d'impulsions ultrasonores ultérieures sont traitées afin de suivre la position temporelle des points de référence provenant de chaque signal d'écho.

Ensuite, suit la phase d'acquisition au cours de laquelle la position temporelle de chaque interface correspondant à chaque écho élémentaire de chaque signal d'écho résultant des impulsions ultrasonores ultérieures à la phase d'acquisition sont suivies et mémorisées. Enfin, une étape d'exploitation se produit au cours de laquelle les données mémorisées dans la phase d'acquisition sont utilisées pour fournir des informations à l'utilisateur tel que l'affichage du diamètre du vaisseau sanguin en fonction du temps.

Enfin, on comprendra que différentes modifications et/ou additions peuvent être réalisées à l'appareil pour la mesure ultrasonore sans s'éloigner de l'étendue de la présente invention définie par les revendications annexées.

## Revendications

1. Appareil de mesure par ultrasons comprenant :
- un transducteur ultrasonore (21) pour émettre une impulsion ultrasonore à une fréquence de répétition prédéterminée vers un objet ayant une pluralité de parois pour recevoir des échos réfléchis à partir desdites parois et pour produire un signal d'écho ayant une pluralité de composants d'échos élémentaires (Eₐₙₜ, Eₚₒₛₜ),
- des moyens de numérisation (27) pour numériser ledit signal d'écho en une série de valeurs numériques,
- une mémoire-tampon (28) pour stocker lesdites valeurs numériques provenant desdits moyens de numérisation (27),
- des moyens de contrôle (29) pour transférer lesdites valeurs numériques provenant desdits moyens de numérisation (27) dans ladite mémoire-tampon (28),
- des moyens de mémorisation (31) pour stocker lesdites valeurs numériques provenant de ladite mémoire-tampon (28), et
- des moyens de traitement (30) pour transférer ladite série de valeurs numériques stockées dans ladite mémoire-tampon (28) dans lesdits moyens de mémorisation (31) entre des impulsions consécutives, caractérisé en ce que
lesdits moyens de traitement (31) sont aptes en outre à prélever à partir des dites valeurs numériques, à des fins de non traitement, un groupe desdites valeurs numériques numérisées entre les composants d'échos élémentaires consécutifs (Eₐₙₜ, Eₚₒₛₜ) devant être traités entre des impulsions consécutives.

2. Appareil de mesure par ultrasons selon la revendication 1, caractérisé en ce que lesdits moyens de traitement (30) sont aptes en outre à retirer ledit groupe de valeurs numériques à partir de ladite série de valeurs numériques avant le stockage desdites valeurs numériques dans lesdits moyens de mémorisation (31).

3. Appareil de mesure par ultrasons selon la revendication 2, caractérisé en ce que
lesdits moyens de traitement (30) sont aptes en outre à retirer ledit groupe de valeurs numériques à partir de ladite série de valeurs numériques avant le stockage desdites valeurs numériques dans ladite mémoire-tampon (28).

4. Appareil de mesure par ultrasons selon l'une des revendication 1 à 3, caractérisé en ce qu'il comprend en outre :
- des moyens d'entrée (35) pour sélectionner ledit groupe de valeurs numériques.

5. Appareil de mesure par ultrasons selon l'une des revendication 1 à 4, caractérisé en ce qu'il comprend en outre :
- des moyens d'affichage (32) pour afficher ladite série de valeurs numériques pour permettre la visualisation (50) dudit signal d'écho, lesdits moyens d'affichage étant aptes à indiquer en outre ledit groupe sélectionné de valeurs numériques (52) avant le retrait desdites valeurs digitales traitées entre deux impulsions consécutives.

## Patentansprüche

1. Ultraschall-Meßgerät, umfassend:
- einen Ultraschallwandler (21) zum Aussenden eines Ultraschall impulses mit einer vorbestimmten Wiederholungsfrequenz auf ein Objekt mit einer Mehrzahl von Wandungen, um von den Wandungen ausgehende Echos zu empfangen, und um ein Echosignal mit einer Mehrzahl von Elementarechokomponenten (Eₐₙₜ, Eₚₒₛₜ) zu erzeugen,
- Digitalisiermittel (27) zum Digitalisieren des Echosignals in eine Serie von numerischen Werten,
- einen Pufferspeicher (28) für das Speichern der von den Digitalisiermitteln (27) kommenden numerischen Werte,
- Steuermittel (29) für das Transferieren der von den Digitalisiermitteln (27) kommenden numerischen Werte in dem Pufferspeicher (28),
- Speichermittel (31) für das Speichern der von dem Pufferspeicher (28) kommenden numerischen Werte,
- Verarbeitungsmittel (30) für das Transferieren der in dem Pufferspeicher (28) gespeicherten Serie von numerischen Werten in die Speichermittel (31) zwischen aufeinanderfolgenden Impulsen,
dadurch gekennzeichnet,
daß die Verarbeitungsmittel (31) ferner ausgebildet sind, um aus den numerischen Werten zwecks Nicht-Verarbeitung eine Gruppe der digitalisierten numerischen Werte zwischen aufeinanderfolgenden Elementarechokomponenten (Eₐₙₜ, Eₚₒₛₜ), die zwischen aufeinanderfolgenden Impulsen zu verarbeiten sind, zu entnehmen.

2. Ultraschall-Meßgerät nach Anspruch 1, dadurch gekennzeichnet, daß die Verarbeitungsmittel (30) ferner ausgebildet sind, um aus der Serie von numerischen Werten die genannte Gruppe von numerischen Werten zurückzuhalten, bevor die numerischen Werte in den Speichermitteln (31) gespeichert werden.

3. Ultraschall-Meßgerät nach Anspruch 2, dadurch gekennzeichnet, daß die Verarbeitungsmittel (30) ferner ausgebildet sind, um aus der Serie von numerischen Werten die genannte Gruppe von numerischen Werten zurückzuhalten, bevor die numerischen Werte in dem Pufferspeicher (28) gespeichert werden.

4. Ultraschall-Meßgerät nach einem der Ansprüche 1 bis 3, dadurch gekennzeichnet, daß es ferner umfaßt:
- Eingabemittel (35) für das Auswählen der Gruppe numerischer Werte.

5. Ultraschall-Meßgerät nach einem der Ansprüche 1 bis 4, dadurch gekennzeichnet, daß es ferner umfaßt:
- Anzeigemittel (32) für das Anzeigen der Serie von numerischen Werten, um die Visualisierung (50) des Echosignals zu ermöglichen, welche Anzeigemittel ausgebildet sind, um ferner die ausgewählte Gruppe numerischer Werte (52) vor der Rücknahme der numerischen Werte anzuzeigen, die zwischen zwei aufeinanderfolgenden Impulsen verarbeitet werden.

## Claims

1. Ultrasonic measuring apparatus comprising :
- an ultrasonic transducer (21) for emitting an ultrasonic pulse at a predetermined repetition frequency towards an object having a plurality of walls, for receiving echos reflected from said walls and for producing an echo signal having a plurality of elementary echo components (Eₐₙₜ, Eₚₒₛₜ),
- digitizing means (27) for digitizing said echo signal into a series of digital values,
- a buffer memory (28) for storing said digital values from said digitizing means (27),
- control means (29) for transferring said digital values from said digitizing means (27) into said buffer memory (28),
- memory means (31) for storing said digital values from said buffer memory (28), and
- processing means (30) for transferring said series of digital values stored in said buffer memory (28) into said memory means (31) between consecutive pulses,
characterized in that
said processing means (31) is further adapted to remove from said digital values, to non-treatment ends, a group of said digital values digitized between consecutive elementary echo components (Eₐₙₜ, Eₚₒₛₜ) to be treated between consecutive pulses.

2. Ultrasonic measuring apparatus according to claim 1, characterized in that
said processing means (30) is further adapted to remove said group of digital values from said series of digital values prior to the storage of said digital values in said memory means (31).

3. Ultrasonic measuring apparatus according to claim 2, characterized in that
said processing means (31) is further adapted to cause the removal of said group of digital values from said series of digital values prior to the storage of said digital values in said buffer memory (28).

4. Ultrasonic measuring apparatus according to one of claims 1 to 3, characterized in that it further comprises :
- user input means (35) for selecting said group of digital values.

5. Ultrasonic measuring apparatus according to one of claims 1 to 4, characterized in that it further comprises :
- display means (32) for displaying said series of digital values to enable visualisation (50) of said echo signal, said display means being adapted to additionally indicate said selected group of digital values (52) prior to removal thereof from said digital values treated between two consecutive pulses.
